(19) Europäisches Patentamt
European Patent Office
Office européen des brevets

(11) **EP 4 070 783 A1**

(12) **EUROPEAN PATENT APPLICATION**
published in accordance with Art. 153(4) EPC

(43) Date of publication:
**12.10.2022 Bulletin 2022/41**

(21) Application number: **19829661.8**

(22) Date of filing: **10.10.2019**

(51) International Patent Classification (IPC):
*A61K 9/00* (2006.01)      *A61K 9/50* (2006.01)
*A61K 31/40* (2006.01)      *A61K 47/10* (2017.01)
*A61K 47/24* (2006.01)      *A61K 47/36* (2006.01)
*A61P 17/00* (2006.01)      *A61P 21/00* (2006.01)

(52) Cooperative Patent Classification (CPC):
(C-Sets available)
A61P 17/00; A61K 9/0014; A61K 9/5015;
A61K 9/5026; A61K 9/5031; A61K 9/5036;
A61K 9/5073; A61K 31/08; A61K 31/366;
A61K 31/728; A61K 45/06; A61K 47/10;
A61K 47/24; A61K 47/36; A61P 21/00      (Cont.)

(86) International application number:
**PCT/ES2019/070689**

(87) International publication number:
**WO 2020/079299 (23.04.2020 Gazette 2020/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2018 ES 201830994**

(71) Applicant: **Pidagema S.L.**
**02002 Albacete (ES)**

(72) Inventor: **MARTÍNEZ CUADRADO, María Pilar**
**02003 Albacete (ES)**

(74) Representative: **Clarke Modet & Co.**
**C/ Suero de Quiñones 34-36**
**28002 Madrid (ES)**

(54) **MICROSPHERES FOR THE TREATMENT OF MUSCULOSKELETAL AND VASCULAR SKIN OR DERMATOLOGICAL DISORDERS**

(57)      Microspheres for the treatment of musculoskeletal and vascular skin or dermatological diseases. The present invention relates to microspheres which are formed by a core comprising a triblock copolymer non-ionic surfactant, a halogenated ether and a glycosaminoglycan, a polyanion layer coating said core and a polycation layer in contact with the external portion of the polyanion layer. Thanks to this combination an effect is achieved which is analgesic, antimicrobial and promotes healing. These microspheres are useful in the treatment of musculoskeletal and vascular skin or dermatological diseases.

FIG. 1

**(Cont. next page)**

(52) Cooperative Patent Classification (CPC): (Cont.)

C-Sets
**A61K 31/08, A61K 2300/00;**
**A61K 31/366, A61K 2300/00;**
**A61K 31/728, A61K 2300/00**

**Description**

FIELD OF THE INVENTION

The present invention belongs to the technical field of compositions for the treatment of different pathologies such as infectious, painful processes with or without a loss of epidermis and/or dermis. In particular, the present invention relates to microspheres and a pharmaceutical composition including microspheres for the treatment of musculoskeletal and vascular skin or dermatological diseases.

BACKGROUND ART

Since ancient times, the administration of medicines has always needed a more or less complex preparation in order to make it possible. Even with the advances caused by the obtaining of chemically pure medicinal substances, which started in the 18th century, it was necessary to equip said substances with a dosage form that would enable the administration thereof in a known and controlled amount (therapeutic dose), via the most suitable route, in a stable, safe and effective manner (SUÑÉ, J. M. Nuevas aportaciones galénicas a las formas de administración [New Galenic contributions to administration forms]. Ferrer Grupo, Barcelona, Spain, 2000, p. 41-47)

One example of new dosage administration forms are microcapsules, microspheres or nanocapsules, which are obtained by microencapsulation of the drug. In said process, a very thin layer made of polymer materials is deposited around the particles of the active ingredient, whether they be solid or liquid. This coating envelops the entire surface of the particle, which acts as a substrate, the external appearance being the same as that of a solid powdery substance.

This type of dosage form has a special relevance in skin diseases or infectious processes in the skin. The document Rodríguez-Llimós, A. C. et al. (2003) describes microspheres made of sodium alginate with the microencapsulation of trans-retinoic acid and salicylic acid, which enable the release of the trans-retinoic acid to be modulated, since it is of dermatopharmaceutical interest to optimise the therapeutic effectiveness and to minimise the secondary reactions, and to protect the skin from the irritating effect of the trans-retinoic acid and salicylic acid, from the deleterious effect of external agents such as air, light and moisture (Rodríguez-Llimós, A. C. et al., Microesferas de alginato para uso dermatofarmacéutico [Alginate microspheres for dermatopharmaceutical use]. Ars pharmaceutica, 2003, vol. 44, No. 3, p. 215-224).

There are many additional examples of the evolution of these microspheres in the state of the art for said uses. One example is the patent ES2363397B1, which discloses multi-layer microspheres, with a polyanion core and which are coated with silver (0). With the inclusion of the silver, an antibacterial and/or antifungal effect is achieved for the treatment of infectious processes, in addition to the advantages presented by a controlled release system such as microspheres.

In this same vein, there is the patent EP16182326A1, which describes microspheres with analgaesic and healing-promoting properties, for which reason it is useful in the treatment and/or prophylaxis of a pain process with deterioration of the cutaneous curing process. Unlike document ES2363397B1, these microspheres comprise sevoflurane, which performs an analgaesic control through the application thereof directly on the ulcer bed, providing immediate, intense and long-lasting analgaesia.

However, none of the documents of the state of the art solves the multiple problems derived from the treatment of skin diseases, musculoskeletal diseases or infectious processes in the skin in one single solution, requiring the application of several treatments to achieve an analgaesic and antimicrobian effect, such that the pain of the user is able to be controlled, at the same time as it favours the curing and healing of the user and prevents possible infections or the reappearance thereof.

DESCRIPTION OF THE INVENTION

The present invention solves the problems present in the state of the art by means of microspheres which are formed by the following combination of layers:

   i) a core comprising:

   -     a triblock copolymer non-ionic surfactant,
   -     a halogenated ether, and
   -     a glycosaminoglycan,

   ii) a polyanion layer coating said core;
   iii) a polycation layer in contact with the external portion of the polyanion layer;

The microspheres of the present invention have a triple function: analgaesic, antimicrobian and healing-promoting, thanks to the combinations of the components thereof.

The present invention provides more clinical safety due to the fact that it keeps the halogenated ether retained in a core and prevents the volatilisation thereof. Furthermore, by being inside of a sustained-release system, the amount of halogenated ether released in a certain time over a certain area can be controlled, which increases the clinical effectiveness.

Moreover, the mixture of the glycosaminoglycan with the surfactant and halogenated ether enhances the healing-promoting effect and care of the epidermis and dermis, added to the powerful analgaesic effect of the halogenated ether.

All of this is added to the fact that the majority of the compounds are biodegradable and biocompatible, making this invention a safe product.

In one aspect of the invention, the triblock copolymer non-ionic surfactant making up the core is a poloxamer. In this manner, the halogenated ether is able to be stabilised in the aqueous environment.

In another aspect of the invention, the halogenated ether forming part of the core is selected from the group comprising isoflurane, methoxyflurane, enflurane, sevoflurane, desflurane and/or mixtures thereof. In a preferred embodiment, the halogenated ether is sevoflurane. The halogenated ethers, in particular sevoflurane, have demonstrated a powerful peripheral analgaesic effect when administered topically.

In another aspect of the invention, the glycosaminoglycan forming part of the core is selected from the group comprising hyaluronic acid, chondroitin sulphate or glucosamine. In a preferred embodiment, the glycosaminoglycan is hyaluronic acid. These compounds favour an ideal equilibrium of the amount of water in the area where there is a loss of continuity of the epidermis, favouring natural debridement and thereby enhancing the healing-promoting effect. In contact with the epidermis, it favours the care, through this equilibrium, of the moisture contributed by the compound.

In another aspect of the invention, the polyanion making up the layer coating the core is selected from the group comprising alginate, lactic acid, polyacrylate, carrageenan, agarose and/or mixtures thereof. In a preferred embodiment, the polyanion is alginate. Thus, the microsphere is able to be obtained and the halogenated ether is retained with a biocompatible and biodegradable material.

In another aspect of the invention, the polycation making up the layer coating the polyanion is selected from the group comprising poly-L-lysine, polyethylene glycol, chitosan, poly-L-ornithine, poly-methylene-co-guanidine and polyethylen-amine and/or mixtures thereof. In a preferred embodiment, the polycation is chitosan. Chitosan is a biodegradable and biocompatible compound which enables the speed with which the halogenated ether and the glycosaminoglycan are released into the external medium to be controlled, apart from achieving a bioadhesive effect of the microsphere.

In another aspect of the invention, the core further comprises at least one statin selected from the group comprising atorvastatin, fluvastatin, lovastatin, pitavastatin, pravastatin, rosuvastatin and simvastatin. In a preferred embodiment, the statin is simvastatin. These statins favour the healing-promoting effect.

In another aspect of the invention, the core further comprises at least one antibiotic selected from the group comprising metronidazole, tinidazole, ornizadole, secnidazole and nimorazole benznidazole, contributing an antimicrobian effect, especially against anaerobic bacteria, controlling the infection in the area to be treated.

In another aspect of the invention, the core further comprises a dermal growth factor which favours the healing-promoting effect.

In another aspect of the invention, the core further comprises at least one serine protease selected from the group comprising chymotrypsin, trypsin or subtilisin granzyme, with the objective of carrying out a chemical debridement of the area in order to be able to enhance the healing-promoting effect.

In another aspect of the invention, the core further comprises collagen. The contribution of this compound has the objective of achieving a carrier effect of the halogenated ether towards deeper layers of the skin such as the dermis, thanks to the triple-helix three-dimensional structure thereof, until it reaches the target point of the pain in joints or in the musculoskeletal insertions. Furthermore, the combination with the ether and the alginate favours the healing-promoting effect by maintaining a moist environment.

In another aspect of the invention, the core further comprises aloe vera (*Aloe Barbandesis*). This compound improves the biochemical, morphological and biomechanical features of the ulcer area by favouring the healing-promoting action. In contact with the epidermis, it favours the care, through this equilibrium, of the moisture contributed by the compound.

In another aspect of the invention, the core further comprises at least one phosphodiesterase inhibitor selected from the group comprising vinpocetine, cilostazol, milrinone, apremilast, mesembrine, moracin M, piclamilast, roflumilast, rolipram, dipyridamole, sildenafil, tadalafil, vardenafil, zaprinast, aminophylline, anagrelide, caffeine, glaucine, paraxanthine, pentoxifylline or theophylline. In a preferred embodiment, the phosphodiesterase inhibitor is sildenafil, tadalafil or vardenafil. The addition of these inhibitors favours vasodilation and therefore the promotion of the healing and care of the skin.

In another aspect of the invention, the microspheres comprise a layer with silver nanoparticles in contact with the external portion of the polcation layer, favouring an ideal environment for controlling microbian growth.

In another aspect of the invention, the microspheres comprise a Lewis acid in contact with the external portion of the polcation, with the advantage of maintaining an ideal environment for inhibiting microbian growth in the affected area.

Another aspect of the invention is related to a pharmaceutical composition comprising the microspheres of the present invention.

The pharmaceutical composition containing the microspheres can be administered in a therapeutically effective amount by any suitable route of administration, such as for example topically, locally or locoregionally. In the context of the invention, therapeutically effective amount is understood as the amount that is sufficient and able to provide a therapeutic or prophylactic effect.

In another aspect, the pharmaceutical composition can be found in the form of a solution, emulsion, paste, gel or aerosol.

Another aspect of the invention is to provide a method for preparing microspheres comprising the following steps:

a) preparing a solution of a polyanion in a concentration of 0.5-4% by weight, a triblock copolymer non-ionic surfactant in a concentration of 0.1-55% by weight, a volatile halogenated ether in a concentration of 0.01-90% by weight and a glycosaminoglycan in a concentration of 0.1 to 10% by weight in distilled water, a polcation solution in a concentration of 0.1-2% by weight in distilled water and a solution comprising at least one divalent cation selected from the group comprising $Be^{+2}$, $Mg^{+2}$, $Cu^{+2}$, $Ca^{+2}$, $Ni^{+2}$, $Sn^{+2}$, $Co^{+2}$, $Fe^{+2}$, $Mn^{+2}$, $Zn^{+2}$ and/or $Ba^{+2}$;
b) stirring by means of magnetic stirring with a speed of 100-1500 rpm the solutions described in step a) in an independent manner,
c) generating microspheres by means of microdroplets of the solution obtained in step a) and putting them in contact with the bivalent cation solution obtained in step a), and
d) putting the microspheres obtained in step c) in contact with the polcation solution obtained in step a).

In another aspect of the invention, the method for preparing microspheres includes an additional step e) wherein silver nanoparticles are added.

In another aspect of the invention, the microspheres are suitable to be used in the treatment of skin and/or dermatological diseases. Preferably, for treating dermatitis.

Skin and/or dermatological diseases are understood as a set of inflammatory or degenerative lesions in the epidermis (pain, itchiness, hypersensitivity, etc.) or a loss of continuity of the epidermis which evolves into deterioration of the solution of continuity and into a loss of substance, epithelium and/or conjunctive mucosa.

In a last aspect of the invention, the microspheres are suitable to be used in the treatment of musculoskeletal diseases.

Skeletomuscular diseases are understood as a set of inflammatory or degenerative lesions of the muscles, tendons, joints, ligaments and nerves. Generally they are located in the vicinity of the neck, back, shoulders, elbows, wrists and hands. The most common medical diagnoses are tendonitis, tenosynovitis, carpal tunnel syndrome, myalgia, neck pain, back pain, etc. The predominant symptom is the pain associated with inflammation, loss of strength and decrease or functional inability of the affected anatomic area.

In a last aspect of the invention, the microspheres are suitable to be used in the treatment of vascular diseases.

Vascular diseases are understood as any condition affecting the circulatory system, which is the system of blood vessels carrying blood from the heart through the rest of the body. This includes diseases in the arteries, veins and lymphatic vessels, and blood disorders that affect circulation.

BRIEF DESCRIPTION OF DRAWINGS

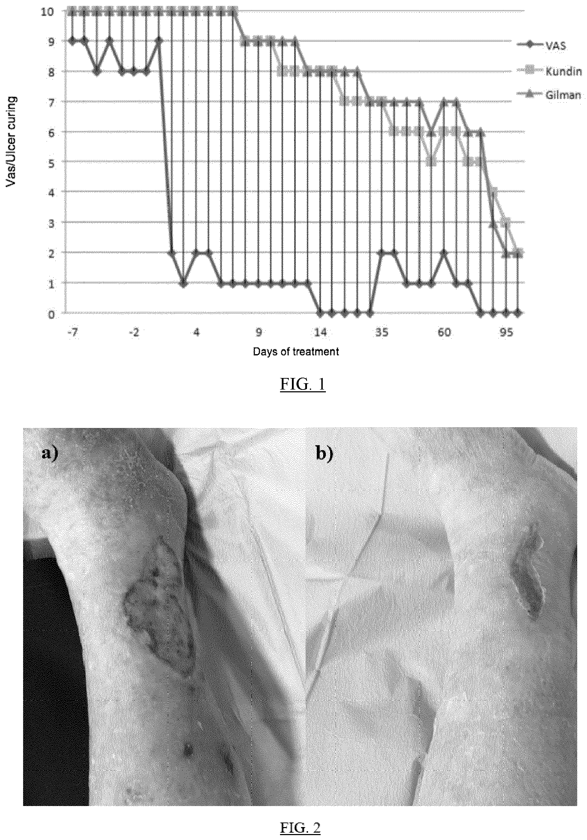

Figure 1. Evolution of the pain by means of a visual analogue scale (VAS) and curing percentage of a vascular ulcer by means of the Kundin and Gilman equation. Microspheres were applied in the affected area, according to one of the preferred embodiments of the invention, characterised in that it is made up of a core formed by poloxamer, sevoflurane and hyaluronic acid, silver coating, alginate (polyanion) and chitosan (polcation).

Figure 2. Images of the evolution in the curing and healing of an ulcer before (a) and after (b) of the application of microspheres in the affected area, according to one of the preferred embodiments of the invention, characterised in that it is made up of a core formed by poloxamer, sevoflurane, hyaluronic acid and simvastatin, alginate (polyanion) and chitosan (polcation).

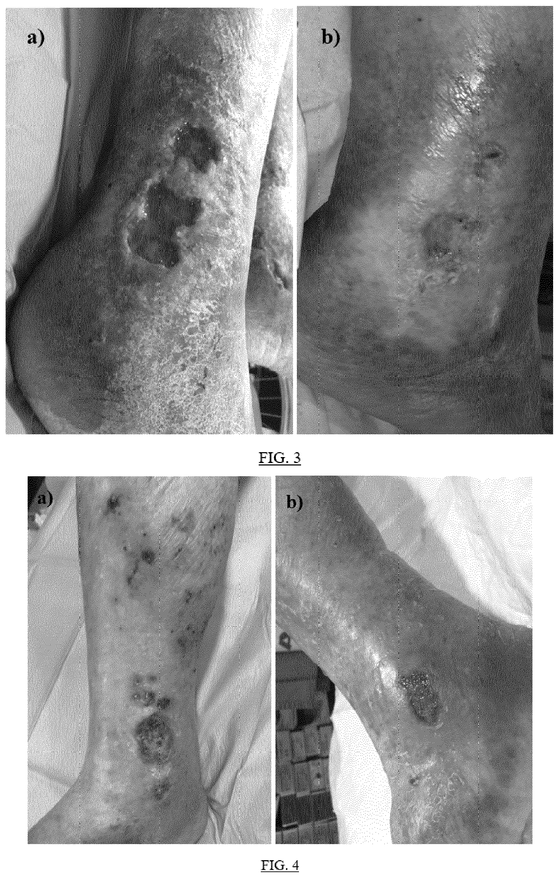

Figure 3. Images of the evolution in the curing and healing of an ulcer before (a) and after (b) of the application of microspheres in the affected area, according to one of the preferred embodiments of the invention, characterised in that it is made up of a core formed by poloxamer, sevoflurane, hyaluronic acid and epidermal growth factor, alginate (polyanion) and chitosan (polycation).

Figure 4. Images of the evolution in the curing and healing of an ulcer before (a) and after (b) of the application of microspheres in the affected area, according to one of the preferred embodiments of the invention, characterised in that they are made up of a core formed by poloxamer, sevoflurane, hyaluronic acid and collagen, alginate (polyanion) and chitosan (polycation).

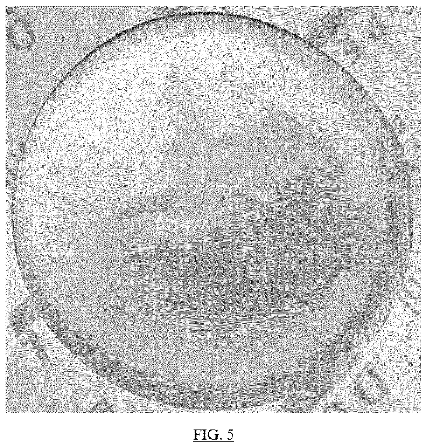

Figure 5. Image of the microspheres of the present invention on a dressing for treating a musculoskeletal disease.

DESCRIPTION OF EMBODIMENTS

Having described the present invention, it is additionally illustrated by means of the following examples.

Example 1. Effectiveness of the microspheres made up of a core formed by poloxamer, sevoflurane and hyaluronic acid, alginate (polyanion) chitosan (polycation) and silver coating in the control of pain, infection and healing in ulcers

A patient was chosen with painful cutaneous ulcers with a high number of reinfections in the right lower limb with 12 months of evolution as a consequence of a spider bite, which were being cured as outpatient with saline irrigations and applications with topical silver (sulphadiazine-argentic ointment or even silver dressings) without success in closing the cutaneous ulcer.

Figure 1 shows the evolution from one week before the aforementioned treatment, representing in negative numbers (-7 to 0) the previous treatment up to the time of the application of one of the preferred embodiments of the microspheres of the invention.

The cultivation of the ulcer was performed in the presence of abundant growths of *P. aeruginosa,* resistant *S. aureusmeticilin* and *E. coli BLEE+* were detected.

Remedies were performed on the ulcer with physiological saline irrigations and subsequently applying on the ulcer bed microspheres in the core of which the sevoflurane and the hyaluronic acid are found, coated with chitosan and followed by a silver dressing every 24 hours.

With regard to antiseptic control, after 7 days of treatment with the microspheres, the cultivation of the ulcer was repeated, with the abundant presence of *P. aeruginosa,* resistant *S. aureusmeticilin* and *E. coli BLEE+* disappearing.

Moreover, as far as the control of the pain, Figure 1 shows the intensity of the pain by means of the VAS , apart from the variation in the measurements of the vascular ulcer with the Kundin scale, calculated as:

$$\text{Ulcer surface} = \text{Length} \times \text{Width} \times 0.875$$

and the Gilman equation calculated as:

$$\text{Linear curing ratio} = \Delta\text{ulcer area / Ulcer perimeter}$$

which measure the curing of the ulcer.

An excellent control of the pain was observed just a few minutes after the application of the microspheres and with a duration of the analgaesic effect of approximately 24 hours with a significant increase in quality of life and an improvement in the healing of the ulcer after the start of the treatment.

The microspheres effectively controlled the infection present in the ulcerous bed of the patient, the pain and reduced the size of the ulcer, the combined synergy of the silver, the halogenated ether and the hyaluronic acid being notable on the effect of reducing the size of the cutaneous ulcer, since in 12 months of evolution the ulcer was not able to be reduced with silver dressings and conventional treatments.

Furthermore, this combination of treatment with microspheres effectively controlled the base nociceptive and neuropathic pain related to the base process, increasing the quality of life of the patient.

Example 2. Effectiveness of the microspheres made up of a core formed by poloxamer, sevoflurane and simvastatin, alginate (polyanion) chitosan (polycation) in healing in ulcers

A patient was selected with vascular ulcers with 24 months of evolution. Every day for 3 weeks, microspheres were applied to it made up of a core formed by poloxamer, sevoflurane, hyaluronic acid and simvastatin, alginate (polyanion) and chitosan (polycation).

Pain was controlled from the initial VAS 7/10 to the final VAS 3/10.

A favourable promotion of the healing was observed, as seen in Figure 2. Said figure shows the images of the evolution in the curing and healing of an ulcer before (a) and after (b) of the application of the composition in the affected area.

Example 3. Effectiveness of the microspheres made up of a core formed by poloxamer, sevoflurane and epidermal growth factor, alginate (polyanion) chitosan (polycation) in healing in ulcers

A patient was selected with vascular ulcers with 24 months of evolution. Microspheres were applied to it every day for 4 weeks, made up of a core formed by poloxamer, sevoflurane, hyaluronic acid and epidermal growth factor, alginate (polyanion) and chitosan (polycation).

A control of the pain was performed from the initial VAS 7/10 to the final VAS 2/10.

A favourable promotion of the healing was observed, as seen in Figure 3, obtained in less than one week. Said figure shows the images of the evolution in the curing and healing of an ulcer before (a) and after (b) of the application of the composition in the affected area.

Example 4. Effectiveness of the microspheres made up of a core formed by poloxamer, sevoflurane and collagen, alginate (polyanion) and chitosan (polycation) in healing in ulcers

A patient was selected with vascular ulcers with 10 months of evolution. Every day for 17 days, microspheres were applied to it made up of a core formed by poloxamer, sevoflurane, hyaluronic acid and collagen, alginate (polyanion) and chitosan (polycation).

Pain was controlled from the initial VAS 8/10 to the final VAS 1/10.

A favourable promotion of the healing was observed, as seen in Figure 4. Said figure shows the images of the evolution in the curing and healing of an ulcer before (a) and after (b) of the application of the composition in the affected area.

Example 5. Effectiveness of the microspheres made up of a poloxamer, sevoflurane, hyaluronic acid and collagen, alginate (polyanion) and chitosan (polycation) for the treatment of a musculoskeletal disease

A patient was selected with a diagnosis of a degenerative musculoskeletal pathology in the trapeziometacarpal joint, stage II, called rhiarthrosis or arthritis of the thumb. The patient was allergic to nonsteroidal anti-inflammatory drugs (NSAIDs), this pharmacological group being the treatment base for this pathology. The patient manages the pain with a brace as a non-pharmacological treatment and 1g of paracetamol every 8 hours as a pharmacological treatment. Even with these measures, the joint has a measured pain, according to the VAS scale, of 8/10, being limited in performing normal daily activities and reducing their quality of life.

A dressing was applied with microspheres (see Figure 5) made up of a core with a pharmaceutical composition of microspheres made up of a core formed by poloxamer, sevoflurane, hyaluronic acid and collagen, alginate (polyanion) and chitosan (polycation).

Pain was controlled from the initial VAS of 8/10 to the final VAS 2/10, in the 10 minutes following the application. The bandage was removed two days after applying it. This pain score was maintained for 5 days with a single administration, the use of paracetamol as rescue medication was suspended, making it possible for the patient to perform normal daily activities and improve their quality of life.

**Claims**

1. Microspheres **characterised in that** each microsphere comprises:

   i) a core comprising:

   - a triblock copolymer non-ionic surfactant,
   - a halogenated ether,
   - a glycosaminoglycan, and
   - a compound selected from among:

      - at least one statin selected from the group comprising atorvastatin, fluvastatin, lovastatin, pitavastatin, pravastatin, rosuvastatin and simvastatin or

- at least one epidermal growth factor, or
- collagen.

ii) a polyanion layer coating said core;
iii) a polycation layer in contact with the external portion of the polyanion layer;

**2.** The microspheres according to claim 1, wherein the triblock copolymer non-ionic surfactant is a poloxamer.

**3.** The microspheres according to any of claims 1 to 2, wherein the halogenated ether is selected from the group comprising isoflurane, methoxyflurane, enflurane, sevoflurane, desflurane and/or mixtures thereof.

**4.** The microspheres according to any of claims 1 to 3, wherein the glycosaminoglycan is selected from the group comprising hyaluronic acid, chondroitin sulphate or glucosamine.

**5.** The microspheres according to any of claims 1 to 4, wherein the polyanion is selected from the group consisting of alginate, lactic acid, polyacrylate, carrageenan, agarose and/or mixtures thereof.

**6.** The microspheres according to any of claims 1 to 5, wherein the polycation is selected from the group comprising poly-L-lysine, polyethylene glycol, chitosan, poly-L-ornithine, poly-methylene-co-guanidine and polyethylen-amine and/or mixtures thereof.

**7.** The microspheres according to any of claims 1 to 6, **characterised in that** they further comprise a layer with silver nanoparticles in contact with the external portion of the polycation layer.

**8.** A pharmaceutical composition comprising microspheres, according to any of claims 1 to 7.

**9.** The composition according to claim 8, **characterised in that** it is in the form of a solution, emulsion, paste, gel or aerosol.

**10.** The microspheres according to any of claims 1 to 7, for use in the treatment of skin and/or dermatological diseases.

**11.** The microspheres according to claim 10, for use in the treatment of skin and/or dermatological diseases wherein the disease is dermatitis.

**12.** The microspheres according to any of claims 1 to 7, for use in the treatment of musculoskeletal diseases.

FIG. 1

FIG. 2

FIG. 3

FIG. 4

FIG. 5

## INTERNATIONAL SEARCH REPORT

| | International application No |
|---|---|
| | PCT/ES2019/070689 |

**A. CLASSIFICATION OF SUBJECT MATTER**

INV.  A61K9/00      A61K9/50      A61K31/40     A61K47/10     A61K47/24
A61K47/36     A61P17/00     A61P21/00

ADD.

According to International Patent Classification (IPC) or to both national classification and IPC

**B. FIELDS SEARCHED**

Minimum documentation searched (classification system followed by classification symbols)

B60D   A61K   A61P

Documentation searched other than minimum documentation to the extent that such documents are included in the fields searched

Electronic data base consulted during the international search (name of data base and, where practicable, search terms used)

EPO-Internal, WPI Data

**C. DOCUMENTS CONSIDERED TO BE RELEVANT**

| Category* | Citation of document, with indication, where appropriate, of the relevant passages | Relevant to claim No. |
|---|---|---|
| X | EP 3 127 538 A1 (FERNANDEZ GINES DAMASO [ES]) 8 February 2017 (2017-02-08) cited in the application paragraph [0025]; claims 1-10 | 1-12 |
| A | EP 3 311 809 A1 (UNIV KAOHSIUNG MEDICAL [TW]) 25 April 2018 (2018-04-25) claim 1 | 1-12 |
| A | WO 2011/086196 A1 (SPHERIC NANOHEALTH S L [ES]; MARTINEZ ESCOBAR SERGIO [ES]) 21 July 2011 (2011-07-21) cited in the application claim 1 | 1-12 |
| X,P | ES 2 692 834 A1 (PIDAGEMA S L [ES]) 5 December 2018 (2018-12-05) claims 1-23 | 1-12 |

☐ Further documents are listed in the continuation of Box C.      ☒ See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 12 March 2020 | 20/03/2020 |

| Name and mailing address of the ISA/ | Authorized officer |
|---|---|
| European Patent Office, P.B. 5818 Patentlaan 2 NL - 2280 HV Rijswijk Tel. (+31-70) 340-2040, Fax: (+31-70) 340-3016 | Konter, Jörg |

Form PCT/ISA/210 (second sheet) (April 2005)

## INTERNATIONAL SEARCH REPORT
Information on patent family members

International application No

PCT/ES2019/070689

| Patent document cited in search report | | Publication date | Patent family member(s) | | Publication date |
|---|---|---|---|---|---|
| EP 3127538 | A1 | 08-02-2017 | EP | 3127538 A1 | 08-02-2017 |
| | | | US | 9554998 B1 | 31-01-2017 |
| EP 3311809 | A1 | 25-04-2018 | CN | 107613972 A | 19-01-2018 |
| | | | EP | 3311809 A1 | 25-04-2018 |
| | | | JP | 2018517703 A | 05-07-2018 |
| | | | US | 2018169136 A1 | 21-06-2018 |
| | | | WO | 2016201682 A1 | 22-12-2016 |
| WO 2011086196 | A1 | 21-07-2011 | ES | 2363397 A1 | 02-08-2011 |
| | | | WO | 2011086196 A1 | 21-07-2011 |
| ES 2692834 | A1 | 05-12-2018 | NONE | | |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2363397 B1

- EP 16182326 A1

**Non-patent literature cited in the description**

- Nuevas aportaciones galénicas a las formas de administración. **SUÑÉ, J. M.** New Galenic contributions to administration forms. Ferrer Grupo, 2000, 41-47

- **RODRÍGUEZ-LLIMÓS, A. C. et al.** Microesferas de alginato para uso dermatofarmacéutico [Alginate microspheres for dermatopharmaceutical use]. *Ars pharmaceutica,* 2003, vol. 44 (3), 215-224